# EUROPEAN PATENT SPECIFICATION

(11) **EP 4 226 913 B1**
(45) Date of publication and mention of the grant of the patent: **09.09.2026**
(21) Application number: 22168917.7
(22) Date of filing: 19.04.2022
(51) Int. Cl.: A61K 9/00, A61K 31/59, A61K 31/593, A61K 33/14, A61P 3/14

(54) **COMPOSITION FOR USE IN SUPPORTING NORMAL BLOOD CALCIUM CONCENTRATIONS IN MAMMALS**
ZUSAMMENSETZUNG ZUR VERWENDUNG IN DER UNTERSTÜTZUNG NORMALER CALCIUMKONZENTRATIONEN IM BLUT VON SÄUGETIEREN
COMPOSITION POUR L'UTILISATION DANS LA MAINTENANCE DES CONCENTRATIONS NORMALES DE CALCIUM DANS LE SANG CHEZ DES MAMMIFÈRES

(30) Priority: 10.02.2022 US 202263308838 P
(43) Date of publication of application: 16.08.2023
(73) Proprietor: Contract Manufacturing Services, LLC, Elmwood, WI 54740 (US)
(72) Inventor: GOFF, Jesse Paul, Elmwood, 54740 (US); SILBERHORN, Tucker James, Elmwood, 54740 (US); HUNDT, Brian Thomas, Elmwood, 54740 (US)
(74) Representative: Schlich

(56) References cited:
- CN-A- 106 689 698
- US-A- 4 931 290
- US-A1- 2012 301 513
- HAJIKOLAEI MOHAMMAD RAHIM HAJI ET AL: "Effect of antepartum vitamin D3 (cholecalciferol) and postpartum oral calcium administration on serum total calcium concentration in Holstein cows fed an acidogenic diet in late gestation", RESEARCH IN VETERINARY SCIENCE, vol. 136, 1 May 2021 (2021-05-01), GB, pages 239 - 246, XP055964357, ISSN: 0034-5288, DOI: 10.1016/j.rvsc.2021.02.017
- MEYER-BINZEGGER M ET AL: "Pharmacokinetics of 1,25-dihydroxyvitamin D3 glycosides from Solanum glaucophyllum extract given in a rumen bolus on blood mineral profiles in dry pregnant dairy cows", RESEARCH IN VETERINARY SCIENCE, BRITISH VETERINARY ASSOCIATION, LONDON, GB, vol. 142, 19 November 2021 (2021-11-19), pages 70 - 77, XP086918833, ISSN: 0034-5288, [retrieved on 20211119], DOI: 10.1016/J.RVSC.2021.11.011

## Description

### BACKGROUND

This disclosure relates to an oral medicament and the administration of such medicament to a periparturient mammal to prevent or treat hypocalcemia in the first days after parturition.

Mammals, and in particular dairy cows, begin lactating after parturition and the onset of lactation can draw more calcium from circulatory blood for placement into milk than the animal's body can replace back into the circulatory blood from dietary calcium and bone calcium reserves. Blood (determined in plasma or serum) calcium concentration in a dairy cow should be about 9 -10 mg/dl (2.25-2.5 mM) in order for calcium to perform its many functions in the body. However, an acute and severe form of hypocalcemia occurs in nearly 5% of multiparous dairy cows as a result of the large and sudden drain of calcium that occurs at the onset of lactation (Goff, 2014). These cows become recumbent and exhibit muscle paresis, a clinical condition known as milk fever, which is often lethal if not appropriately treated. About 50% of multiparous dairy cows and 25% of heifers experience a significant degree of hypocalcemia that can be referred to as sub-clinical hypocalcemia around the time of calving. The serum calcium concentration below which a cow is said to have significant hypocalcemia varies within the literature from 8.0 to 8.6 mg/dl (2 to 2.15 mM). Though hypocalcemia can be treated with intravenous or oral calcium administration, cows with significant hypocalcemia during the first days of lactation are at increased risk of immune dysfunction, metritis, displacement of the abomasum, retained placenta, mastitis, and ketosis (Goff, 2014).

Fortunately, cattle and all mammals have a system that generally allows them to restore normal blood calcium concentrations within a few days after calving. Calcium homeostasis is mediated primarily by the parathyroid gland, which secretes parathyroid hormone (PTH) in response to any reduction in blood calcium concentration. PTH stimulates release of calcium from bone calcium stores and reduces the amount of calcium lost to urine. PTH also activates the renal enzyme that produces the vitamin D hormone, 1,25-dihydroxyvitamin D. The 1,25-dihydroxyvitamin D stimulates transcellular absorption, also known as active transport, of calcium across the intestinal epithelium to greatly increase the utilization of dietary calcium (Goff, 2018). When calcium homeostasis is working properly the cow experiences only a small and short-lived decline in blood calcium concentration during the periparturient period. Unfortunately, in about half of the cows in a herd, the ability to maintain blood calcium concentration above about 8 mg/dl (2 mM) in the first few days of lactation is impaired. A reduced ability of bone and kidney cells to respond to PTH stimulation has been implicated as the defect in calcium homeostasis that results in prolonged or severe hypocalcemia (Goff, 2014). Several factors can interfere with calcium homeostasis, causing more drastic and longer lasting declines in blood calcium concentration. Cow factors affecting calcium homeostasis include advancing age of the cow and breed.

Nutritional factors also influence the resilience of the calcium homeostasis system. Dietary cations, such as potassium (K⁺), sodium (Na⁺), calcium (Ca⁺²), and magnesium (Mg⁺²), will raise the pH of the blood of the cow if they are absorbed into the blood of the cow. Dietary anions, such as chloride (Cl⁻), sulfate (SO4⁻²) and phosphate (PO4⁻³), will acidify the blood of the cow if they are absorbed into the blood. Cations or anions in the diet that are not absorbed have no impact on blood pH. The difference in the number of milliequivalents of cations and anions absorbed from the diet helps determine blood pH (Goff, 2018). Cows are typically in a state of compensated metabolic alkalosis as their diet consists of forages that are typically high in K⁺. Since K⁺ is absorbed from the diet with nearly 100% efficiency, forage K⁺ is strongly alkalinizing. Na⁺ is also highly alkalinizing as diet or water Na⁺ is also absorbed with nearly 100% efficiency. Ca⁺² and Mg⁺² cations are absorbed from the diet with much lower efficiency than K⁺ or Na⁺, but they can be present in diets in relatively high concentrations so that absorbed Ca⁺² and Mg⁺² will contribute to the alkaline state of the blood of the cow.

Cows in a state of compensated metabolic alkalosis as a result of being fed forages high in K⁺ do not respond to PTH stimulation as well as cows placed in a state of compensated metabolic acidosis by addition of anions to their diet (Goff, 2014). Metabolic alkalosis impairs bone calcium resorption (Block, 1994). In addition, the ability of PTH to stimulate timely production of 1,25-dihydroxyvitamin D is impaired, reducing the utilization of dietary calcium. Adjusting diet cation-anion difference to induce a compensated metabolic acidosis is a commonly utilized means of reducing milk fever and hypocalcemia in dairy cows. Adding chloride and sulfate anions to the pre-calving diet of the cow can greatly reduce the degree of hypocalcemia the cow will experience at calving. This restores the sensitivity of bone and kidney cells to PTH which improves calcium homeostasis mechanisms. The details of nutritional management of the diet fed before and after calving to reduce hypocalcemia in dairy cows is well described (Goff, 2014). Diets with anions added can be unpalatable and can depress diet dry matter intake prior to calving. Reducing diet consumption around the time of parturition can increase susceptibility of the cow to ketosis, metritis, and displacement of the abomasum. There are, however, caveats to the use of anions to reduce hypocalcemia. If the addition of anions to the diet fails to acidify the cow enough to cause urine pH to be below about 6.8 the effectiveness of the diet to reduce hypocalcemia is greatly reduced. If urine pH is depressed below about 5.75 the dry matter intake may be depressed and lead to other metabolic problems associated with reduced feed consumption prior to calving. While proper anion supplementation can improve calcium homeostasis, some sub-clinical hypocalcemia and even clinical hypocalcemia (milk fever) can still occur.

In many cows that suffer from hypocalcemia after calving it seems the calcium homeostasis response is delayed from that of cows that do not suffer appreciable subclinical or clinical hypocalcemia. This is particularly evident in cows that develop relapsing cases of clinical hypocalcemia (Goff et al., 1989). The traditional treatment of the cow with milk fever is to administer 10-12 grams of calcium intravenously to rapidly, though temporarily, restore blood calcium concentrations to levels that will support normal calcium functions, such as nerve and muscle function. This medical intervention supports blood calcium concentrations for 6-10 hours which often provides enough time for the cow's calcium homeostasis mechanisms to be activated. Upon full activation of the calcium homeostasis mechanisms, the cow obtains enough calcium from the bone and from the diet to support normal blood calcium concentrations while continuing to lose calcium to milk production. However, in some cows the calcium homeostasis mechanisms are so impaired that the cow relapses and has a second or third bout of milk fever 12-24 hours after the calcium injection. As Goff et al., (1989) demonstrated, these cows are very slow to produce 1,25-dihydroxyvitamin D. Once they begin to produce 1,25-dihydroxyvitamin D, they gain control of calcium homeostasis and are able to maintain blood calcium concentrations with no further treatment. Cows exhibiting earlier elevations of blood 1,25-dihydroxyvitamin D concentrations after calving have an improved ability to maintain normal blood calcium concentrations than cows that do not increase blood 1,25-dihydroxyvitamin D promptly after calving. Other studies have demonstrated that the peak of blood 1,25-dihydroxyvitamin D concentration is lower in the normocalcemic cows that produce 1,25-dihydroxyvitamin D promptly after calving than in cows producing 1,25-dihydroxyvitamin D later, since in the latter cows the 1,25-dihydroxyvitamin D is made in response to more severe hypocalcemia. (Goff et al., 1991). These and other studies caused many researchers to study the administration of 1,25-dihydroxyvitamin D as a means of preventing hypocalcemia in dairy cows.

Administration of 1,25-dihydroxyvitamin D or its analogs to dairy cows prior to calving has been successfully used to reduce hypocalcemia (Horst et al., 1997). Unfortunately, it has the disadvantage that prediction of calving time is critical to its success. If a single dose of 1,25-dihydroxyvitamin D or its analogs is administered orally or parenterally less than 12-24 hours before calving or is administered alone after calving the 1,25-dihydroxyvitamin D will not stimulate active transport of calcium quickly enough to impact hypocalcemia in many of the cows. If given too early before calving (more than 3 to 4 days), a second and even third dose may have to be administered to be effective. Using analogs of 1,25-dihydroxyvitamin D with longer plasma half life can extend the therapeutic window by 1-2 days but still must be administered at least 12-24 hours before calving to be effective. Prolonged release formulations of 1,25-dihydroxyvitamin D have been described, but still must be administered prior to calving to be effective. Administering small doses daily until and even after the cow calves can also effectively prevent hypocalcemia (Horst et al., 1997). However, the prolonged exposure to exogenous 1,25-dihydroxyvitamin D can inhibit the dairy cow's ability to make endogenous 1,25-dihydroxyvitamin D, so that she may develop hypocalcemia 4 to 10 days after the effects of the exogenous 1,25-dihydroxyvitamin D have waned. Larger doses of 1,25-dihydroxyvitamin D can extend the effective window slightly to perhaps 5-7 days prior to calving, but this also exacerbates the problems associated with prolonged exposure to exogenous 1,25-dihydroxyvitamin D (Horst et.al., 1997). The issues of timing of administration and possibility of inducing inhibition of endogenous 1,25-dihydroxyvitamin D synthesis greatly diminish the practicality of use of 1,25-dihydroxyvitamin D to prevent periparturient hypocalcemia.

A second approach to maintaining more normal blood calcium concentrations in periparturient cows has been to administer orally a large dose of readily solubilized calcium shortly after calving. Generally, 30-75 g calcium is administered in the form of a bolus, paste or liquid drench. By raising the concentration of ionized calcium in the fluid (rumen fluid or intestinal chyme) directly in contact with absorptive cells above about 4 mM, the calcium can be absorbed utilizing a passive paracellular transport mechanism (Goff, 2018). This mechanism is independent of stimulation by 1,25-dihydroxyvitamin D. This causes a rapid but limited increase in blood calcium concentration. It has been demonstrated that oral administration of 50 g calcium in the form of readily solubilized calcium chloride caused about 4 g calcium to be absorbed into the blood of the cows, resulting in an increase of blood calcium of approximately 1 mg/dl (0.25 mM) within 1 hour which lasted about 6-8 hours. Larger doses can be given but increase the therapeutic window (length of time blood calcium is increased toward normal) by only 1-3 hours (Goff and Horst, 1993). The general approach to increase the therapeutic window is to administer a second or possibly third dose of oral calcium at 12-24 hours after the first dose, which is generally administered within hours of calving. This entails more labor for the farmer to locate and restrain the dairy cow and administer each dose. It can also sometimes cause excessive absorption of chloride or sulfate; the anions of the more commonly used calcium salts. This can cause the cow to enter a state of uncompensated metabolic acidosis which can impose further health challenges to the cow (Goff and Horst, 1994). While only a small portion of administered calcium is absorbed by the paracellular route, the remainder is eligible for absorption once the active calcium transport mechanisms have been activated by endogenous production of 1,25-dihydroxyvitamin D.

US 4 931 290 A discloses *inter alia* a milk fever prophylactic composition orally administerable to a dairy cow, the composition comprising at least about 150 grams of a water-soluble calcium salt of an organic acid, and at least one magnesium compound which can additionally comprise Vitamin D₃.

### SUMMARY

This disclosure describes a composition for oral administration to a periparturient mammal at risk of developing hypocalcemia within 0-6 hours after parturition; the composition comprising a form of calcium rapidly absorbable by the periparturient mammal using passive paracellular transport across the intestinal epithelium and a 1-alpha hydroxylated vitamin D compound in an amount sufficient to stimulate active transport of calcium across the intestinal epithelium, the calcium and the 1-alpha hydroxylated vitamin D being administered concurrently to support maintenance of normal blood calcium concentrations in the periparturient mammal. The composition according to the invention is defined in claim 1. The combination according to the invention is defined in claim 7.

Any subject-matter beyond the scope of the claims does not form part of the invention and is provided for comparison and/or reference purposes only.

This disclosure further describes that the form of calcium administered comprises, alone or preferably in combination, readily water soluble calcium salts such as calcium chloride, calcium sulfate, calcium propionate, calcium acetate, calcium lactate, or calcium formate.

This disclosure further describes wherein calcium chloride is incorporated in an amount sufficient to induce a compensated metabolic acidosis within 8 hours of administration to support normal sensitivity of tissues to parathyroid hormone.

This disclosure further describes that the 1-alpha hydroxylated vitamin D compound comprises 1,25-dihydroxyvitamin D or 1-alpha hydroxyvitamin D or analogs thereof in an amount sufficient to stimulate transcellular rumen and/or intestinal absorption of calcium.

This disclosure further describes that the 1-alpha hydroxylated vitamin D compound is a glycoside of 1,25-dihydroxyvitamin D as found in calcinogenic plants or extracts prepared from the calcinogenic plants.

This disclosure further describes that the 1-alpha hydroxylated vitamin D compound is not incorporated in an amount so great that it causes significant inhibition of the renal 25-hydroxyvitamin D-1-alpha hydroxylase enzyme, thereby inhibiting endogenous production of 1,25-dihydroxyvitamin D, such that delayed hypocalcemia occurs 4-10 days after administration of the composition.

This disclosure further describes the composition wherein the 1-alpha hydroxylated vitamin D compound and the calcium are administered concurrently in the form of a bolus, tablet, or pellet with a density of at least 1.2 kg / L (g/ml) and released from the bolus, tablet or pellet over a time period that is less than 2 hours.

This disclosure also describes a method for increasing the level of calcium in blood or for normalizing blood calcium levels or for maintaining normal or healthy calcium levels in blood or for avoiding hypocalcemia of a mammal; the method comprises administering 1-alpha hydroxylated vitamin D compound and a readily soluble calcium source concurrently to a periparturient mammal shortly after parturition.

This disclosure also describes a method wherein the 1-alpha hydroxylated vitamin D compound and the calcium are administered concurrently in the form of a bolus, tablet or pellet with a density of at least 1.2 kg / L (g/ml) and released from the bolus, tablet or pellet over a time period that is less than 2 hours.

This disclosure further describes a method wherein the 1-alpha hydroxylated vitamin D compound and the calcium salts of the compound are able to support more normal blood calcium concentrations when administered concurrently in a single dose without any additional doses.

This disclosure further describes a method wherein the composition is able to support normal blood calcium concentrations or reduce hypocalcemia if administered to a periparturient mammal within 6 hours of giving birth, and most preferred when administered within 3 hours of giving birth.

This disclosure further describes a method for reducing hypocalcemia in a periparturient mammal at risk of developing hypocalcemia at the onset of lactation, such as the dairy cow.

This disclosure further describes a method wherein the form of calcium administered includes calcium chloride or calcium sulfate in an amount sufficient to induce a compensated metabolic acidosis in the animal to support sensitivity of the tissues to parathyroid hormone so as to improve calcium homeostasis within eight hours of administration.

This disclosure further describes a method wherein the form of calcium is, alone or preferably in combination, calcium chloride, calcium sulfate, calcium propionate, calcium acetate, calcium lactate, or calcium formate in an amount sufficient to promote passive, vitamin D-independent, paracellular absorption of calcium to support normal blood calcium concentrations and reduce hypocalcemia during the first 6 to 12 hours following administration without inducing an uncompensated metabolic acidosis in the animal.

This disclosure further describes a method wherein the 1-alpha hydroxylated vitamin D compound comprises 1,25-dihydroxyvitamin D or 1-alpha hydroxyvitamin D or analogs thereof administered in an amount sufficient to stimulate transcellular rumen and/or intestinal absorption of calcium to support normal blood calcium concentrations or reduce hypocalcemia from 12 hours to 72 hours following administration.

This disclosure further describes a method wherein the 1-alpha hydroxylated vitamin D compound is a glycoside of 1,25-dihydroxyvitamin D obtained from calcinogenic plants or extracts prepared from the calcinogenic plants administered in an amount sufficient to stimulate transcellular rumen and/or intestinal absorption of calcium to support normal blood calcium concentrations or reduce hypocalcemia from 12 hours to 72 hours following administration.

This disclosure further describes a method wherein the 1-alpha hydroxylated vitamin D compound is not incorporated in an amount so great that it causes significant inhibition of the renal 25-hydroxyvitamin D-1-alpha hydroxylase enzyme, thereby inhibiting endogenous production of 1,25-dihydroxyvitamin D, such that delayed hypocalcemia occurs 4-10 days after administration of the composition.

### BRIEF DESCRIPTION OF THE DRAWINGS

Figure 1 is a graphical view showing increase in plasma 1,25-dihydroxyvitamin D by 4 hours after receiving the bolus.
Figure 2 is a graphical view showing blood calcium levels post calving comparing treated cows and nontreated cows.
Figure 3 is a graphical view showing blood calcium levels post calving comparing treated cows and nontreated cows.
Figure 4 is a graphical view showing blood calcium levels post calving comparing treated cows and nontreated cows.
Figure 5 is a graphical view showing plasma calcium levels after calving.
Figure 6 is a graphical view showing plasma calcium levels after treatment and no treatment.
Figure 7 is a graphical view showing plasma calcium levels after treatment and no treatment.
Figure 8 is a graphical view showing plasma calcium levels after treatment and no treatment.

### DETAILED DESCRIPTION

This disclosure describes a method of providing a readily soluble source of oral calcium salts to promote rapid paracellular absorption of calcium and which acidify the cow to enhance tissue sensitivity to parathyroid hormone, together with exogenous 1,25-dihydroxyvitamin D or 1-alpha hydroxylated analogs thereof to promote more prolonged and more efficient transcellular absorption of dietary calcium to prevent or mitigate hypocalcemia and milk fever. The method preferably providing the soluble source of calcium salts and exogenous 1,25-dihydroxyvitamin D or analogs thereof shortly after parturition. The soluble source of calcium salts and exogenous 1,25-dihydroxyvitamin D are provided only once. By shortly after parturition is meant that the soluble source of calcium salts and exogenous 1,25-dihydroxyvitamin D or analogs thereof are provided within 6 hours following parturition.

The terms 1,25-dihydroxyvitamin D and 1,25-OH2D will be used interchangeably herein and should be considered as referring to the same structure.

The basic structure of 1,25-OH2D comprises a secosteroid. The carbons of this secosteroid are numbered by convention as described in Maestro et al., (2019). Numerous other possible vitamin D compounds that are 1-alpha hydroxylated could be utilized in addition or in lieu of 1,25-OH2D, including but not limited to those listed in Maestro et. al., (2019), but, according to the invention only the 1-alpha hydroxylated vitamin D compounds as defined in the claims are encompassed.

Unless specifically implied to the contrary, "vitamin D" without a subscript, used alone, as a suffix or prefix, or as a modifier, refers to any of vitamin D₂ (ergocalciferol), vitamin D₃ (cholecalciferol), vitamin D₄ (22-dihydroergocalciferol), and vitamin D₅ (sitocalciferol).

Vitamin D secosteroids that are hydroxylated on carbon number 1 in the alpha configuration will be referred to as "1-alpha hydroxylated vitamin D" compounds. The 1-alpha hydroxylated vitamin D compounds include 1,25-dihydroxyvitamin D compounds (i.e., 1,25-dihydroxyvitamin D₂, 1,25-dihydroxyvitamin D₃, 1,25-dihydroxyvitamin D₄, and 1,25-dihydroxyvitamin D₅); active analogs thereof; or inactive analogs thereof that increase the blood, tissue, or cellular level of a 1,25-dihydroxyvitamin D compound or an active analog thereof.

"Analogs," used with reference to 1-alpha hydroxylated vitamin D compounds, refers to biological precursors of 1,25-dihydroxyvitamin D compounds, biological metabolites of 1,25-dihydroxyvitamin D compounds, or any natural or synthetic compound recognized in the art as having a structural similarity to-or being derived from-1,25-dihydroxyvitamin D compounds. "Active" used with reference to 1-alpha hydroxylated vitamin D compounds and analogs thereof refers to those analogs that directly produce a vitamin D-dependent effect in a target tissue or target cell without being modified or further metabolized by a non-target tissue, non-target cell, or other site elsewhere in the body. When used to modify "1-alpha hydroxylated vitamin D", the term "active" refers to 1,25-dihydroxyvitamin D compounds or active analogs thereof. An "inactive" analog of a 1-alpha hydroxylated vitamin D compound also useful to this invention is one that is not directly able to produce a vitamin D-dependent effect in a target tissue or target cell without being modified or further metabolized by a non-target tissue, non-target cell, or other site elsewhere in the body. "Vitamin D-dependent effects" include any of the effects disclosed herein, known in the art, or hereafter discovered that result from administration or treatment of 1,25-dihydroxyvitamin D compounds. Examples of vitamin D-dependent effects include, without limitation, stimulation of transcellular calcium absorption across the rumen and intestinal epithelium and anabolic and catabolic actions on bone.

Biologically inactive analogs of 1,25-dihydroxyvitamin D that are acted upon within the body to form biologically active 1,25-dihydroxyvitamin D or analogs thereof may be used. They are generally less expensive to utilize than 1,25-dihydroxyvitamin D. One such compound is 1-alpha vitamin D3, which becomes hydroxylated at the carbon 25 position to form active 1,25-OH2D3.

The amount of a 1-alpha hydroxylated vitamin D compound required to be effective for maintenance of normal blood calcium concentration or reducing hypocalcemia will, of course, vary with the individual mammal being treated and is ultimately at the discretion of the veterinary practitioner or animal husbandman. The factors to be considered include the nature of the formulation, the mammal's body weight, surface area, age, general condition, and the particular compound to be administered. In general, a suitable effective dose of 1,25-dihydroxyvitamin D3 equivalent activity is in the range of about 0.1 to about 2 µg/kg body weight, preferably 0.2 -1 ug/kg, and most preferred 0.25- 0.5 ug/kg.

The exogenous 1-alpha hydroxylated vitamin D and the calcium salt are preferably administered to the mammal in a preferred form of a bolus. For purposes of this application a bolus comprises a rounded mass typically cylindrical in shape with a density greater than 1.2 g/ml to ensure it does not float on the rumen raft of a ruminant. Any shape is within the scope of this disclosure that is suitable for administration to the particular mammal being treated. Also, for purposes of this application, the term mammal as used herein shall apply to all livestock such as all bovines, buffaloes, hogs, horses, mules, donkeys, sheep, and goats. Of especial interest are dairy cows. The term "ruminant" refers to an animal which has a digestive system which comprises a rumen such as in the family of Bovidae including but not limited to domestic cattle, bison, buffalo, sheep, goats, antelopes, and other species within the bovidae family; deer and allied species; camels, llamas and their related relatives, as defined by Honacki et al, Mammal Species of the World, 1982.

Numerous documents in the literature, as summarized in Horst et al., (1997), describe the use of 1-alpha hydroxylated compounds administered prior to parturition as a means of preventing or reducing the degree of hypocalcemia an animal might experience at the onset of lactation. These compounds are effective because they stimulate the transcellular or active transport of calcium across the intestinal tract and may also enhance bone calcium release. Use of vitamin D compounds for control of hypocalcemia has not been widely adopted due to three problems. These are timing of administration of the vitamin D compound relative to parturition, possibility of causing toxicity from excessive hypercalcemia, and the possibility of causing inhibition of endogenous synthesis of 1,25-dihydroxyvitamin D, causing hypocalcemia to occur some 4-10 days after the administered vitamin D compound's effects have waned. The timing of administration of prior art required the vitamin D compound be administered within a window of a few days before calving to be effective. The 1-alpha vitamin D compounds of the prior art had to be given at least 12-24 hours prior to calving as the 1-alpha vitamin D requires 12 to 24 hours to initiate transcription and translation of proteins involved in the active transport of calcium across intestinal epithelium. For example, if 1-alpha hydroxyvitamin D3 or 1,25-dihydroxyvitamin D3 are administered more than four days prior to parturition they are not effective as the beneficial effects of these compounds on active transport of calcium will have waned before the cow calves. It can be very difficult to accurately predict that a cow is going to calve between 24 and 96 hours after administration of the 1-alpha hydroxylated vitamin D compound. Since this is difficult, prior art generally recommended that a second dose be administered if the cow did not calve within 4-5 days of the first dose being administered (Horst et. al., 1997). Larger doses or use of more potent analogs of 1,25OH2D can extend the window of effectiveness by 1-3 days, but can also cause problems associated with acute hypercalcemia and toxicity. Complicating this is a third problem. As described in Horst et al., (1997), repeated dosing or administration of high doses of vitamin D compounds to prevent hypocalcemia and milk fever can result in substantial inhibition of endogenous synthesis of 1,25-dihydroxyvitamin D. This can result in development of hypocalcemia some 4-10 days after the beneficial effects of the exogenous vitamin D compounds have waned.

According to the teaching of the document US Pat. No. 9,757,415 a composition comprising Solanum glaucophyllum glycosides for preventing and/or treating hypocalcemia and for stabilizing blood calcium levels in dairy cows can be produced which does not cause excessive hypercalcemia or cause excessive inhibition of the endogenous synthesis of 1,25-dihydroxyvitamin D. The composition utilizes glycosides of 1,25-OH2D3 derived from solanum glaucophyllum leaves as a source of a 1-alpha hydroxylated vitamin D compound that will be converted to 1,25-OH2D3 by rumen microbial cleavage of the glycoside(s) liberating 1,25-OH2D3. An advantage of this approach is that the leaf material is not expensive and can be considered a natural forage source of a 1-alpha vitamin D compound. Calcium in the form of dolomite is mixed with solanum glaucophyllum glycoside to form a hard compressed bolus that is only slowly solubilized in the rumen fluids providing a more prolonged release of solanum glaucophyllum glycoside into the rumen fluids. To be most effective the composition of this patent must be administered between 24 and 72 hours prior to calving. The calcium in the form of dolomitic limestone is not readily soluble in rumen fluid and cannot be absorbed to any great extent by the vitamin D independent paracellular pathway across the rumen and intestinal epithelium. Therefore, the dolomitic calcium will not contribute substantially to maintenance of normal blood calcium concentrations prior to activation of the transcellular calcium absorption pathways initiated by the 1,25-OH2D liberated from the 1,25-OH2D glycosides contained within the S glaucophyllum leaf. The composition could be administered up to 72 hours prior to calving, which is similar to a priori art. Since prediction of the time of parturition can be difficult in the cow, should the cow fail to calve within 5 days of administration of the slow-release bolus a second bolus may be administered. The major obstacle to use of this preparation is, as with most of the a priori art involving use of vitamin D compounds to prevent periparturient hypocalcemia, accurate timing of administration.

According to the teaching of document US Pat. No. 5,395,622 a bolus comprised of calcium salts, including calcium chloride and calcium sulfate can support improved blood calcium concentrations in periparturient cows for 4-8 hours after administration to a cow. Therefore, as taught by US Pat. No. 5,395,622 the administration of a second bolus of calcium salts is encouraged at 12 or 24 hours after administration of the first bolus to improve blood calcium concentration over a longer period. Goff and Horst (1993) demonstrated that the length of time the blood calcium concentrations will be increased following an oral dose of calcium can be extended 1-4 hours by increasing the dose of calcium administered. However, the dose must be limited as hypercalcemia could develop. More importantly, if large doses of calcium chloride providing more than 3-3.5 equivalents of chloride are utilized the cow risks development of uncompensated metabolic acidosis. As demonstrated in Goff and Horst, (1994), calcium propionate is also a readily soluble source of calcium but it is not acidifying. Calcium propionate may also extend the length of time the dose is able to elevate blood calcium concentrations when compared to calcium chloride (Goff and Horst, 1993).

The preventive approach of this disclosure has several advantages over the approaches of the prior art. The bolus can be given as soon as the farmer observes that the cow has delivered a calf. The farmer does not have to try to predict the correct time to administer the exogenous 1-alpha hydroxylated vitamin D to the cow prior to calving. In addition, the preferred bolus form of this disclosure is administered as a single dose, obviating the necessity to find and restrain the cow to give multiple doses of an oral calcium supplement to promote normocalcemia as is often recommended when utilizing currently available calcium boluses. These advantages are achieved by provision of a mixture of soluble calcium salts such as calcium chloride and calcium propionate to promote passive absorption of calcium across the rumen and intestinal epithelium to maintain blood calcium concentrations for up to 12 hours after administration of the preparation. This provides the time necessary for the exogenous 1-alpha hydroxylated vitamin D in the preparation to stimulate the active transport of calcium across rumen and intestinal tissues which will support more normal blood calcium concentrations for the next 60-72 hours. The oral calcium salts include calcium chloride and are given in such a way as to promote a small degree of metabolic acidosis, which may promote bone calcium release and endogenous synthesis of 1,25-dihydroxyvitamin D to support normocalcemia. By also including calcium propionate or another non-acidifying readily absorbable calcium source in the preparation a larger total dose of calcium can be safely administered so that more normocalcemic blood calcium concentrations can be maintained for 12 or more hours after administration. The exogenous administration of 1-alpha hydroxylated vitamin D will raise blood 1,25-dihydroxyvitamin D concentrations rapidly, in less than 4 hours, which begins the process of activating active transcellular calcium transport 4-12 hours before the endogenous synthesis of 1,25-dihydroxyvitamin D would normally be expected to occur in response to the onset of hypocalcemia. The exogenous 1-alpha hydroxylated vitamin D allows greater use of diet calcium and any remaining bolus calcium residing in the lumen of the intestinal tract. This synergistic effect of oral soluble calcium salts and exogenous 1-alpha hydroxylated vitamin D compounds allows greater efficacy against periparturient hypocalcemia than either prior art approach alone and with greater ease of use.

The amount of calcium salt administered should be sufficient to support normal blood calcium concentrations for up to 12 hours. This protects the cow from hypocalcemia until the exogeneous 1-alpha hydroxylated vitamin D has had sufficient time to stimulate the more efficient active transport of calcium from the intestinal lumen into the blood of the cow. The amount of 1,25-dihydroxyvitamin D should be sufficient to stimulate intestinal calcium absorption within 12-18 hours of administration. Prior art compositions had to be administered prior to calving to be effective, so they typically included doses of 1-alpha hydroxylated vitamin D compounds in the range of 300 - 600 ug total to ensure adequate blood levels of 1,25-dihydroxyvitamin D activity would be available throughout the period of time (24-96 hours) they might be administered prior to calving so it could stimulate active calcium transport for the first few days following calving. Because the composition of this invention is administered at a readily identifiable time point, within 6 hours of calving, the amount of 1-alpha hydroxylated vitamin D of this invention found to be effective is less than 300 ug total dose. This reduces the risk of inhibition of the renal 25-hydroxyvitamin D-1-alpha hydroxylase enzyme which can reduce endogenous synthesis of 1,25-dihydroxyvitamin D, as has been observed to cause hypocalcemia to develop 4-10 days after administration of larger doses of 1-alpha hydroxylated vitamin D compounds.

### METHODS OF PRODUCTION

The methods described below utilize amounts of calcium and 1-alpha-hydroxylated vitamin D compounds found to be most effective for the dairy cow weighing from 500 to 700 kg. Doses for other species would have to be adjusted to accommodate differing body weight

### Preparation of a Solid Dose Oral Preparation (i.e. Bolus)

In this most preferred preparation for administration to cattle, the calcium salts (preferably 80-150 g CaCl2·xH2O (where x is equal to or greater than 0 and equal to or less than 6) and up to 250 g calcium propionate) and 1-alpha hydroxylated vitamin D compounds (preferably equivalent to 100-280 ug 1,25-dihydroxyvitamin D) are mixed together, along with other materials that may include but are not limited to, compounds that support gluconeogenesis (i.e. propylene glycol, glycerin, propionate), electrolyte balance (i.e. potassium and sodium chloride, magnesium sulfate and chloride), yeast, non-steroidal anti-inflammatory drugs, and rumen fermentable feedstuff (i.e. alfalfa meal, soybean meal). These materials are mixed together with water until a pumpable mixture is formed. It is also possible to add water to a mixing vessel and add the above ingredients to the water and mix until a homogenous pumpable mixture is formed. Regardless of the preparation method utilized to make the pumpable mixture, the pumpable mixture can then be utilized to fill a mold made from paper, polymer, metal or other material that will result in the shape of an item that is able to be swallowed by the animal (i.e. pill or bolus). Upon filling the mold with the homogenous pumpable mixture, the mold will be allowed to rest for a period of 0.5 - 24 hours (preferably under cooling) until the homogenous pumpable mixture has solidified. Following solidification, the solid product may or may not be coated with a mixture to allow for easier swallowing of the product. The resulting product will be packaged in a way to protect it from breakage and exposure to the elements. Product will be administered to an animal orally. US Patent 5,395,622 and US Published patent Application US20070098810 describe in detail the manufacture of a bolus and both are hereby incorporated by reference in their entirety.

For ease of administration to the cow the effective doses of the calcium salts and the exogenous 1-alpha hydroxylated vitamin D compounds may be distributed into one large bolus or multiple smaller boluses.

### Preparation of a Tablet

In this version the calcium salts (preferably 80-150 g calcium chloride and up to 250 g calcium propionate) and 1-alpha hydroxylated vitamin D compounds (preferably equivalent to 100-280 ug 1,25-dihydroxyvitamin D) are mixed together, along with materials that are commonly utilized in the manufacture of compression tablets or compression boluses. These materials are termed excipients and may include items such as binders (i.e. dextrose, microcrystalline cellulose), lubricants (i.e. magnesium stearate), flow agents (i.e. dicalcium phosphate, silicone dioxide), disintegrants (i.e. starch), and other excipients. This mixture is then subjected to sufficient force to cause the free-flowing material to be compressed into one solid mass. Once a solid mass has been created any number of these units may be delivered to an animal utilizing an applicator and technique that is known to individual skilled in the art of veterinary medicine or animal husbandry. For ease of administration to the cow the effective doses of the calcium salts and the exogenous 1-alpha hydroxylated vitamin D compounds may be distributed into one large tablet or multiple smaller tablets.

### Preparation of a drench product

In this version the calcium salts (preferably 80-150 g calcium chloride and up to 250 g calcium propionate) and 1-alpha hydroxylated vitamin D compounds (preferably equivalent to 100-280 ug 1,25-dihydroxyvitamin D) are mixed together, along with other materials that may include but are not limited to, compounds that support gluconeogenesis (i.e. propylene glycol, glycerin, propionate), electrolyte balance (i.e. potassium and sodium chloride, magnesium sulfate and chloride), yeast, non-steroidal anti-inflammatory drug, and rumen fermentable feedstuff (i.e. alfalfa meal, soybean meal). These materials are mixed together and packaged as dry material with about 85 to 90% dry matter. When utilizing 1,25-dihydroxyvitamin D or similar lipid soluble vitamin D compounds as the form of 1-alpha hydroxylated vitamin D, it will be necessary to incorporate an agent to keep the water insoluble 1,25-dihydroxyvitamin D in solution. Many such materials are known in the arts and include agents such as medium chain triglycerides to form an emulsion with the 1,25-dihydroxyvitamin D vitamin D keeping it suspended in the water of the drench. If the source of 1-alpha hydroxylated vitamin D is a glycoside or glucuronide, as might be contained in material derived from calcinogenic plants such as Solanum glaucophyllum, an emulsifying agent is not necessary as these vitamin D compounds are already water soluble. On farm, the drench mix is added to a suitable amount of water (typically 0.5-20 liters) in a bucket or other suitable vessel and administered orally to the cow shortly after calving via a drench gun, esophageal tube, or tube that extends to the rumen as commonly practiced in large animal veterinary medicine.

### Preparation of gels or pastes

In this case the calcium salts (preferably 80-120 g calcium chloride and 100-250 g calcium propionate) and 1-alpha hydroxylated vitamin D compounds (preferably equivalent to 100 to 280 ug of 1,25-dihydroxyvitamin D) are mixed with carriers (i.e. propylene glycol, glycerol, water, or vegetable oils) and thickeners (i.e. xanthan gum, silicon dioxide) to form an emulsion or suspension that is placed into a tube, typically in a volume between 250 and 400 ml. Other compounds may also be included as described above. The tube carrying the mixture is typically designed to fit into a caulking gun for oral administration into the back of the mouth of the cow using techniques familiar to those practiced in the art of animal husbandry and veterinary medicine. For ease of administration to the cow the effective doses of the calcium salts and the exogenous 1-alpha hydroxylated vitamin D compounds may be distributed into one large paste tube or multiple smaller tubes.

The following examples are intended as illustrations.

### EXAMPLES

### Example 1

Calcium salt boluses were prepared that incorporated either 150 ug 1,25-dihydroxyvitamin D3 or 5, 7.5 or 10 g of leaf from solanum glaucophyllum, a plant that contains a glycoside form of 1,25-dihydroxyvitamin D3 in its leaves. Within the cow's rumen the 1,25-dihydroxyvitamin D3 glycoside, which is biologically inert, is cleaved by rumen bacterial enzymes to bioactive 1,25-dihydroxyvitamin D3. The batch of leaf material used for this experiment, and all of the following experiments described in this document, was determined to contain the equivalent of 14 ug 1,25-dihydroxyvitamin D3 / g leaf utilizing a modification of the method of Gil et al., (2007). The Holstein cows utilized for this study were pregnant and near the end of their lactation and were to be dried off within 2 weeks of the study. These cows were utilized as they should have been in positive calcium balance and therefore would be expected to have relatively low concentrations of endogenous 1,25-dihydroxyvitamin D in their blood allowing better resolution of increases in plasma 1,25-dihydroxyvitamin D that could be attributed to the administration of the boluses. Though our target animal is the cow immediately after calving, previous studies have determined plasma 1,25-dihydroxyvitamin D concentrations can be quite variable (low and very high) depending on the extent of hypocalcemia the cow has experienced prior to calving and time of sampling of the blood. The cows weighed from 600-720 kg and were producing from 16 to 28 kg milk / day. Treatment consisted of administering 2 of the boluses of a single type to a cow so that a cow received boluses comprised of either 300 ug of 1,25-dihydroxyvitamin D, or boluses comprised of 10, 15, or 20 g of S. glaucophyllum leaf in total, supplying the equivalent of 140, 210, and 280 ug 1.25-dihydroxyvitamin D activity. Blood samples were collected into lithium heparin vacutainer tubes from the jugular vein of each cow just before the boluses were administered (time 0) and 1, 4, 24, 48, 72, and 96 hours after the boluses were administered. Plasma was analyzed for concentration of 1,25-dihydroxyvitamin D3 by liquid chromatography-mass spectrometry.

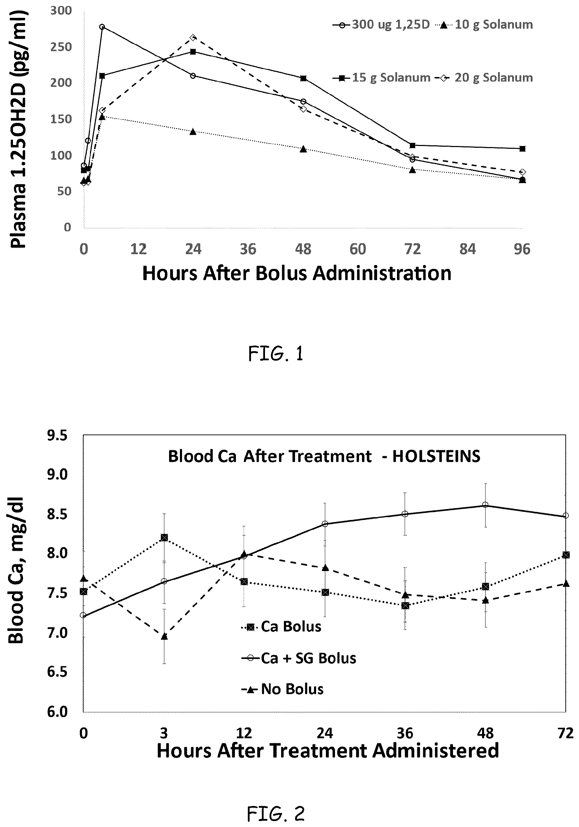

As shown in Figure 1, all cows had a significant increase in plasma 1,25-dihydroxyvitamin D by 4 hours after receiving the bolus. Plasma 1,25-dihydroxyvitamin D concentrations remained significantly elevated above pre-treatment 1,25-dihydroxyvitamin D concentration at 48 hours after administration but not at 72 or 96 hours after administration. Plasma 1,25-dihydroxyvitamin D rose more quickly in cows that received synthetic 1,25-dihydroxyvitamin D3 than in cows receiving the 1,25-dihydroxyvitamin D3 glycoside from the solanum glaucophyllum leaf. This may reflect the time it takes for the rumen bacteria to convert the glycoside to the active 1,25-dihydroxyvitamin D so that it may be absorbed into the blood.

For examples 2 thru 5, statistical analysis consisted of repeated measures analysis of variance with cow nested within treatment and time after bolus administration as the repeated measure. Treatment plasma calcium means were compared at each time point using Tukey's test of comparison of means and differences are declared to be significant when the probability of the null hypothesis being correct is P < 0.075.

### Example 2

Example 2 was performed on a commercial dairy with both Jersey and Holsteins fed a pre-calving diet with a high anion inclusion rate designed to help control hypocalcemia. Only multiparous cows were utilized and cows assigned to a treatment were blocked by lactation number. The effects of treatments on each breed are presented separately and then combined. The treatment protocol was identical for Jersey and Holstein cows and the cows were housed together for the final 21 days prior to calving.

### Holsteins

The average urine pH of Holsteins during the 2 weeks prior to calving was 5.68. The dry matter (DM) intake of the cows was estimated at 23 lbs. (10.5 kg) / day in these cows prior to calving. Seven Holsteins were not administered any boluses at calving. Nine cows received a commercial oral calcium bolus, supplying about 50 g calcium, primarily from calcium chloride. Those Holstein cows received the treatment at calving and again the following morning (12-24 hrs. after the first bolus). Ten Holstein cows were treated with 2 calcium salt boluses which also contained solanum glaucophyllum leaf a single time within 2 hours of calving. These two boluses supplied 78 g calcium total, the majority from calcium chloride with some from calcium propionate and 14 g solanum glaucophyllum leaf, supplying 1-alpha hydroxylated vitamin D in the form of glycosides of 1,25-dihydroxyvitamin D determined to be equivalent to 196 ug 1,25-dihydroxyvitamin D3. Plasma samples were obtained from each cow prior to treatment, and 3, 12, 24, 36, 48, and 72 hours after treatment. Plasma calcium concentration was determined using the Arsenazio III reagent method.

Figure 2 presents Holstein cow average blood Ca ± the standard error of that average. In Holsteins receiving the Ca + SG Bolus, plasma calcium was significantly greater at 36 hours and 48 hours after calving than in cows receiving No Bolus or the Ca Bolus. Both Ca containing boluses significantly increased plasma calcium concentrations 3 hours after calving compared to Holstein cows receiving No Bolus treatment. Only Ca + SG Bolus Holstein cows experienced mean plasma Ca above 8.25 mg/dl (2.06 mM) during the study. No Bolus and Ca Bolus treatment groups average plasma calcium concentration failed to reach even 8.0 mg/dl (2mM) during the first 3 days after calving.

### Jerseys

The average urine pH of the Jersey cows prior to calving was 5.85 and 25% of the Jerseys had urine pH below 5.5. Dry matter feed intake prior to calving was estimated at 18 lbs. (8.2 kg) DM / day. Ten Jersey cows received a commercial oral Ca Bolus at calving and a second Ca Bolus 12-24 hours later. Fourteen Jersey cows received the 2 Ca + SG Boluses with calcium and Solanum glaucophyllum leaf at calving only. Three Jersey cows received no treatment (No Bolus) after calving. Bolus composition and timing of blood samples was as described above for the Holsteins of this example

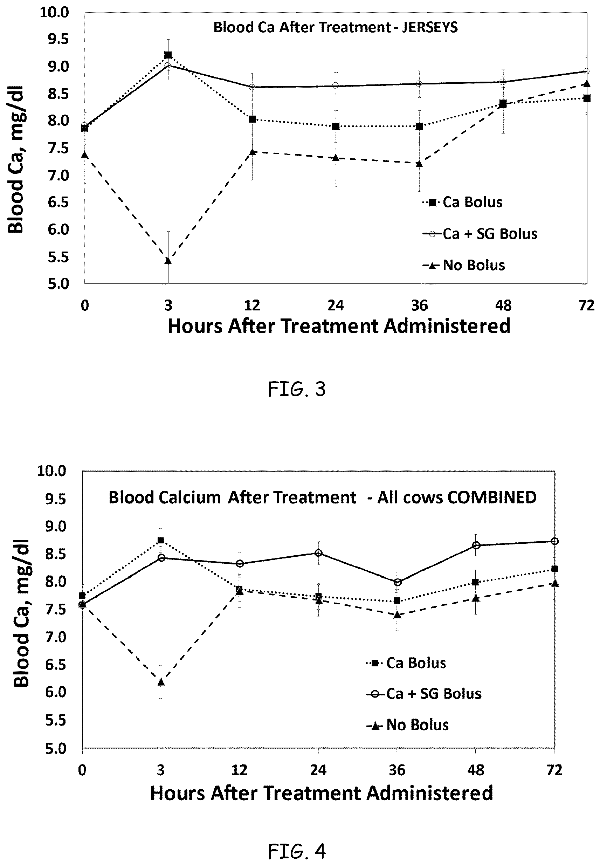

As illustrated in Figure 3, at the 3-hour time point both boluses, Ca + SG Bolus and Ca Bolus treated cows exhibited significantly increased plasma calcium compared to cows receiving No Bolus. From that point on the Ca Bolus and No Bolus cows had similar plasma calcium concentration. The cows getting the Ca + SG Bolus had significantly higher plasma calcium than cows getting No Bolus at 3,12, 24 and 36 hrs. after calving. Plasma calcium of cows receiving the Ca + SG Bolus was statistically better than in Ca Bolus cows at 24 and 36 hours after calving. Only the Ca + SG Bolus cows had mean plasma calcium above 8.5 mg/dl between 12 and 72 hours of the study.

### Combined Jerseys and Holsteins Data

Since all the cows were similarly housed and fed before and after calving, the results from all cows of Example 2 are combined in Figure 4. With increased numbers of cows at each time point the statistical power of the study is increased. The Ca + SG Bolus treatment group has greater plasma calcium than the NO bolus treatment group from 3 thru 48 hours after calving and has greater plasma calcium concentration than the Ca Bolus treatment group at 24, 36 and 48 hours after calving. Only the Ca + SG Bolus cows achieved mean plasma calcium concentration above 8.5 mg/dl between 12 and 72 hours.

### Example 3

In Example 3, cows from a herd located on a farm were fed anions in their diet to achieve urine pH between 6 and 6.8. This is generally considered an effective means of preventing clinical hypocalcemia in the dairy cow. The milk production of this herd was 103 lbs. (46.8 kg) / day with 3.9 % fat. The average urine pH of these cows the weeks before calving was 6.6. In this study nine multiparous cows got a single commercial calcium bolus (Ca Bolus) at calving (43 g Ca / bolus) and again 12-24 hours after calving, providing a total of 86 g Ca primarily from calcium chloride. Six cows received 2 Ca + 10 g SG Boluses at calving only. These 2 boluses were comprised of 78 g calcium primarily from calcium chloride and calcium propionate and 10 g solanum glaucophyllum leaf total supplying the equivalent of 140 ug of 1,25-dihydroxyvitamin D as the glycoside of 1,25-dihydroxyvitamin D3. Seven cows received 2 Ca + 15 g SG Boluses at calving only. These 2 boluses were comprised of 78 g calcium primarily from calcium chloride and calcium propionate and contained 15 g solanum glaucophyllum leaf supplying the equivalent of 210 ug of 1,25-dihydroxyvitamin D as the glycoside of 1,25-dihydroxyvitamin D3. Plasma samples were obtained from the jugular vein prior to bolus administration (Time=0) and 4, 12, 24, 48, and 72 hours after calving.

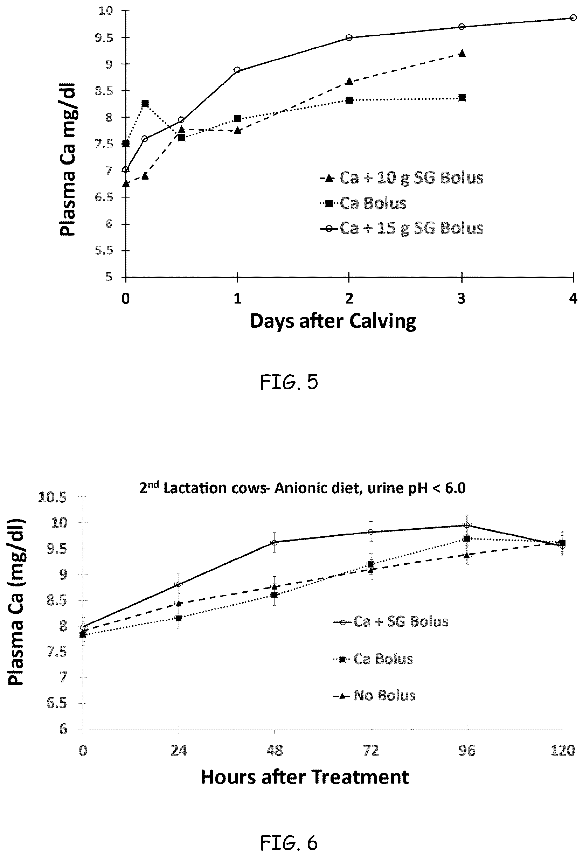

As illustrated in Figure 5, the Ca Bolus cows had slightly higher plasma calcium concentration prior to any treatment than the cows of either of the Ca + SG Bolus treatments. In Ca + 15 g SG Bolus cows the plasma calcium concentration was above 9 mg/dl by day 2. The Ca Bolus cows had plasma calcium concentration below 8.5 mg/dl the first 3 days after calving. Cows in the Ca + 10 g SG group had plasma calcium exceed 8.5 mg/dl by day 2. These data suggested a dose of 15 g solanum glaucophyllum leaf was more effective than a dose of 10 g solanum glaucophyllum leaf for incorporation into the bolus to prevent hypocalcemia.

### Example 4

This example utilized multiparous Holstein and Holstein X Jersey crossbred cows on a large commercial dairy farm feeding anions to prevent milk fever and hypocalcemia. Urine pH averaged 5.7 in the weeks prior to calving.

Sixty cows were assigned to one of three treatments based on expected calving date and blocked by lactation number and breed (so there were nearly equal numbers of Holsteins or Holstein X Jersey cows and cows entering their 2nd (N=10) or 3rd and greater lactation (N=10) in each treatment group. The treatments were: A. Two calcium with solanum glaucophyllum leaf boluses at calving only (supplying 78 g calcium and 1,25-dihydroxyvitamin D3 glycoside equivalent to 196 ug 1,25-dihydroxyvitamin D). B. Gelatin capsule boluses containing calcium, comprised of calcium chloride supplying 40 g calcium / dose. These were administered at calving and again 12-24 hours after calving, for a total of 80 g calcium / cow. C. Cows receiving No Bolus after calving. Plasma samples were obtained shortly after calving and prior to treatment (Time=0) and at 24, 48, 72, 96, and 120 hours after calving.

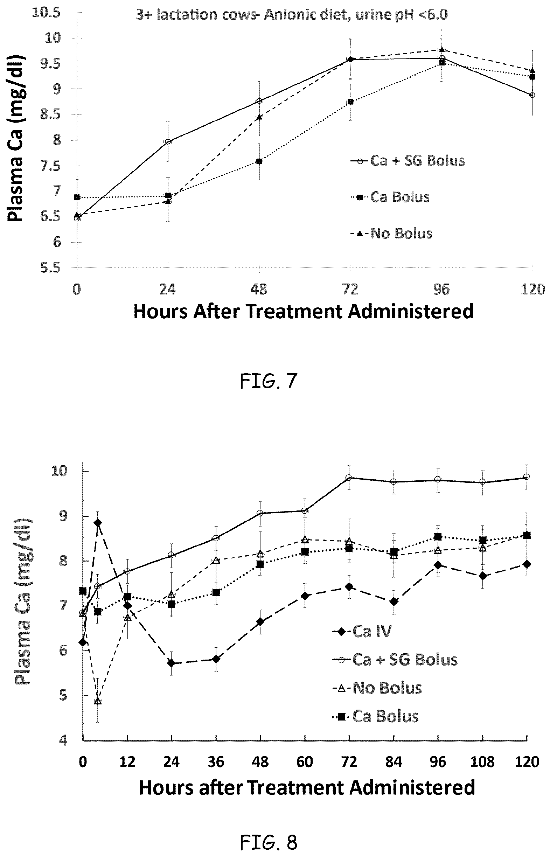

The data are presented based on the lactation number the cow was entering. Each treatment group had 10 cows in it. In 2^{nd} lactation cows (Figure 6), cows in the Ca + SG Bolus treatment group had greater blood Ca concentration than either of the other treatments from 24-96 hours after treatment. Figure 7 presents plasma calcium concentration in cows entering their 3rd and later lactation. Mean plasma calcium concentrations above 8 mg/dl were attained within 24 hours in the Ca + SG Bolus treatment group. The 3^{rd} or greater lactation cows in the No Bolus group had plasma calcium concentration above 8 mg/dl at 48 hours after treatment. The 3^{rd} or greater lactation cows that received the Ca Bolus attained a plasma calcium concentration by 72 hours after treatment.

### Example 5

In Example 5, multiparous cows were utilized from several small farms that were not utilizing an anionic diet to reduce the incidence of hypocalcemia in the cows. Within a few hours of calving, cows were administered one of four treatments. Six cows were not treated with any bolus after calving (No Bolus). Four cows were treated with intravenous calcium (10.5 g calcium in the form of calcium gluconate) after calving (IV Ca). Twenty-two cows received 2 boluses after calving that contained calcium salts and solanum glaucophyllum leaf, supplying 78 g calcium and 1,25-dihydroxyvitamin D3 glycoside equivalent to 196 ug 1,25-dihydroxyvitamin D3 (Ca + SG Bolus). Twenty-four cows were treated at calving and again 12-24 hrs. later with commercial oral calcium salt boluses that supplied 40- 44 g calcium each for a total calcium dose of 80-88 g, primarily from calcium chloride (Ca Bolus).

All cows had plasma calcium concentration determined prior to treatments being applied and all would be considered to be hypocalcemic (plasma calcium below 8.0 mg/dl). Cows that received No Bolus treatment after calving exhibited a further decline in blood calcium during the first four hours after calving. As illustrated in Figure 8, in the cows that received No Bolus blood calcium concentration remained below 8.0 mg/dl (commonly considered to be indicative of a cow with subclinical hypocalcemia) until nearly 96 hours after calving. Cows in the Ca Bolus treatment group exhibited a small decline in calcium at the four-hour time point and their blood calcium concentration at the 4-hour time point was significantly better than in cows receiving No Bolus. However, from 12- 120 hours after calving their plasma calcium concentrations were similar to those of cows receiving No Bolus after calving. Administration of calcium solution intravenously (Ca IV) resulted in the highest plasma calcium concentration observed at four hours after calving. However, from 24 to 84 hours after calving these cows had the lowest plasma calcium concentration. The intravenous calcium administration inhibited the calcium homeostasis mechanisms of the cow, causing more hypocalcemia to be observed on days 2 and 3 following lactation than if no treatment had been given. Cows in the Ca + SG Bolus treatment exhibited a rise in plasma calcium concentration at each time point after calving and their blood calcium concentration went above the threshold for sub-clinical hypocalcemia of 8.0 mg/dl by 24 hrs. after calving. From 4 hours after treatment thru 84 hours after treatment, cows receiving the Ca + SG Bolus at calving only had significantly higher plasma Ca concentration than cows receiving the commercial oral calcium boluses given two times after calving.

## Claims

1. A composition for use in a method for treating hypocalcemia or preventing hypocalcemia in a periparturient mammal at risk of developing hypocalcemia, the composition comprising:
a readily soluble calcium source rapidly absorbable by the periparturient mammal using passive paracellular transport across the intestinal epithelium, wherein the readily soluble calcium source comprises calcium chloride, calcium sulfate, calcium propionate, calcium acetate, calcium lactate, or calcium formate, alone or in combination; and
a 1-alpha hydroxylated vitamin D compound in an amount sufficient to stimulate active transport of calcium across the intestinal epithelium of the periparturient mammal, wherein the 1-alpha hydroxylated vitamin D compound is 1-alpha hydroxylated 1,25-dihydroxyvitamin D, 1-alpha hydroxylated 1-alpha hydroxyvitamin D, or 1-alpha hydroxylated glycosides of 1,25-dihydroxyvitamin D, and
wherein the method comprises orally administering the composition to the periparturient mammal concurrently within 0-6 hours after parturition to support normal blood calcium concentrations in the periparturient mammal.

2. The composition for use according to claim 1, wherein the readily soluble calcium source is calcium chloride and the readily soluble calcium source induces a compensated metabolic acidosis within 8 hours of administration to support normal sensitivity of tissues to parathyroid hormone.

3. The composition for use according to claim 1 or claim 2, wherein the periparturient mammal is a ruminant mammal and the 1-alpha hydroxylated vitamin D compound is in an amount sufficient to stimulate active transport of calcium across the rumen or intestinal epithelium of the periparturient mammal.

4. The composition for use according to any one of the preceding claims, wherein the 1-alpha hydroxylated vitamin D has a β-glycoside linkage and wherein a source of the 1-alpha hydroxylated vitamin D compound with the β-glycoside linkage is *Solanum glaucophyllum, Cestrum diurnum, Trisetum flavescens*, or combinations thereof.

5. The composition for use according to any one of the preceding claims, wherein an amount of the 1-alpha hydroxylated vitamin D compound is less than 300 ug as a total dose such that the total dose does not cause significant inhibition of the renal 25-hydroxyvitamin D-1-alpha hydroxylase enzyme, nor inhibit endogenous production of 1,25-dihydroxyvitamin D, and thus prevents delayed hypocalcemia from occurring 4-10 days after administration of the composition.

6. The composition for use according to any one of the preceding claims, wherein the 1-alpha hydroxylated vitamin D compound and the readily soluble calcium source are administered concurrently in the form of a bolus, tablet, or pellet with a density of at least 1.2 kg/L (g/ml) and released from the bolus, tablet or pellet over a time period less than 2 hours.

7. A combination for use in a method for increasing or normalizing blood calcium levels in a mammal with hypocalcemia, the method comprising:
concurrently orally administering the combination to a periparturient mammal within 6 hours after parturition, wherein the combination separately comprises a 1-alpha hydroxylated vitamin D compound and a readily soluble calcium source,
wherein the 1-alpha hydroxylated vitamin D compound is 1,25-dihydroxyvitamin D, 1-alpha hydroxyvitamin D, or glycosides of 1,25-dihydroxyvitamin D, and
wherein the readily soluble calcium source comprises calcium chloride, calcium sulfate, calcium propionate, calcium acetate, calcium lactate, or calcium formate, alone or in combination.

8. The combination for use according to claim 7, wherein the combination is in the form of a bolus, tablet or pellet with a density of at least 1.2 kg/L (g/ml) and the 1-alpha hydroxylated vitamin D compound and the readily soluble calcium source are completely released from the bolus, tablet or pellet over a time period less than 2 hours.

9. The combination for use according to claim 7 or claim 8, wherein the 1-alpha hydroxylated vitamin D compound and the readily soluble calcium source are provided in a single total dose without any additional doses to support more normal blood calcium concentrations.

10. The combination for use according to any one of claims 7 to 9 for use in a method for supporting normal blood calcium concentrations or reducing hypocalcemia, wherein the combination is administered to a periparturient mammal within 3 hours after giving birth.

11. The combination for use according to any one of claims 7 to 10 for use in a method for reducing hypocalcemia in a periparturient mammal at risk of developing hypocalcemia at the onset of lactation, wherein the periparturient mammal is a dairy cow.

12. The combination for use in a method for increasing or normalizing blood calcium levels in a mammal with hypocalcemia according to any one of claims 7 to 11, wherein administering the readily soluble calcium source promotes passive, vitamin D independent, paracellular absorption of calcium to support normal blood calcium concentrations and reduce hypocalcemia during the first 6-12 hours following administration of the combination and without inducing an uncompensated metabolic acidosis in the periparturient mammal, and
wherein the readily soluble calcium source includes calcium chloride or calcium sulfate in an amount sufficient to induce a compensated metabolic acidosis in the periparturient mammal to support sensitivity of the tissues to parathyroid hormone so as to improve calcium homeostasis within 8 hours after administration.

13. The combination for use in a method for increasing or normalizing blood calcium levels in a mammal with hypocalcemia according to any one of claims 7 to 12, wherein the periparturient mammal is a ruminant mammal, and wherein administering the 1-alpha hydroxylated vitamin D compound stimulates active transport of calcium across the rumen and/or intestinal epithelium to support normal blood calcium concentrations or reduce hypocalcemia from 12-72 hours following administration.

14. The combination for use according to any one of claims 7 to 13, wherein the 1-alpha hydroxylated vitamin D has a β-glycoside linkage and wherein a source of the 1-alpha hydroxylated vitamin D compound with the β-glycoside linkage is *Solanum glaucophyllum. Cestrum diurnum, Trisetum flavescens*, or combinations thereof.

## Patentansprüche

1. Zusammensetzung zur Verwendung in einem Verfahren zum Behandeln von Hypocalciämie oder Verhindern von Hypocalciämie bei einem perinatalen Säugetier, bei dem das Risiko besteht, Hypocalciämie zu entwickeln, wobei die Zusammensetzung umfasst:
eine leicht lösliche Calciumquelle, die von dem perinatalen Säugetier unter Verwendung eines passiven parazellulären Transports durch das Darmepithel schnell absorbiert werden kann, wobei die leicht lösliche Calciumquelle Calciumchlorid, Calciumsulfat, Calciumpropionat, Calciumacetat, Calciumlactat oder Calciumformiat allein oder in Kombination umfasst; und
eine 1-alpha-hydroxylierte Vitamin-D-Verbindung in einer ausreichenden Menge, um den aktiven Transport von Calcium durch das Darmepithel des perinatalen Säugetiers zu stimulieren, wobei die 1-alpha-hydroxylierte Vitamin-D-Verbindung 1-alpha-hydroxyliertes 1,25-Dihydroxyvitamin D, 1-alpha-hydroxyliertes 1-alpha-Hydroxyvitamin D oder 1-alpha-hydroxylierte Glycoside von 1,25-Dihydroxyvitamin D ist, und
wobei das Verfahren das gleichzeitige orale Verabreichen der Zusammensetzung an das perinatale Säugetier innerhalb von 0-6 Stunden nach der Geburt umfasst, um normale Blutcalciumkonzentrationen bei dem perinatalen Säugetier zu unterstützen.

2. Zusammensetzung zur Verwendung gemäß Anspruch 1, wobei die leicht lösliche Calciumquelle Calciumchlorid ist und die leicht lösliche Calciumquelle innerhalb von 8 Stunden nach Verabreichung eine kompensierte metabolische Azidose induziert, um die normale Empfindlichkeit von Geweben gegenüber Parathormon zu unterstützen.

3. Zusammensetzung zur Verwendung gemäß Anspruch 1 oder Anspruch 2, wobei das perinatale Säugetier ein Wiederkäuer ist und die 1-alpha-hydroxylierte Vitamin-D-Verbindung in einer Menge vorliegt, die ausreicht, um den aktiven Transport von Calcium durch das Pansen- oder Darmepithel des perinatalen Säugetiers zu stimulieren.

4. Zusammensetzung zur Verwendung gemäß einem der vorhergehenden Ansprüche, wobei das 1-alpha-hydroxylierte Vitamin D eine β-Glycosidbindung aufweist und wobei eine Quelle der 1-alpha-hydroxylierten Vitamin-D-Verbindung mit der β-Glycosidbindung *Solanum glaucophyllum, Cestrum diurnum, Trisetum flavescens* oder Kombinationen davon ist.

5. Zusammensetzung zur Verwendung gemäß einem der vorhergehenden Ansprüche, wobei eine Menge der 1-alpha-hydroxylierten Vitamin-D-Verbindung weniger als 300 µg als Gesamtdosis beträgt, so dass die Gesamtdosis keine signifikante Hemmung des renalen 25-Hydroxyvitamin D-1-alpha-Hydroxylase-Enzyms verursacht oder die endogene Produktion von 1,25-Dihydroxyvitamin D hemmt und somit verhindert, dass 4-10 Tage nach Verabreichung der Zusammensetzung eine verzögerte Hypocalciämie auftritt.

6. Zusammensetzung zur Verwendung gemäß einem der vorhergehenden Ansprüche, wobei die 1-alpha-hydroxylierte Vitamin-D-Verbindung und die leicht lösliche Calciumquelle gleichzeitig in der Form eines Bolus, einer Tablette oder eines Pellets mit einer Dichte von mindestens 1,2 kg/l (g/ml) verabreicht und über einen Zeitraum von weniger als 2 Stunden aus dem Bolus, der Tablette oder dem Pellet freigesetzt werden.

7. Kombination zur Verwendung in einem Verfahren zur Erhöhung oder Normalisierung des Blutcalciumspiegels bei einem Säugetier mit Hypocalciämie, wobei das Verfahren umfasst: gleichzeitiges orales Verabreichen der Kombination an ein perinatales Säugetier innerhalb von 6 Stunden nach der Parturition, wobei die Kombination getrennt eine 1-alpha-hydroxylierte Vitamin-D-Verbindung und eine leicht lösliche Calciumquelle umfasst,
wobei die 1-alpha-hydroxylierte Vitamin-D-Verbindung 1,25-Dihydroxyvitamin D, 1-alpha-Hydroxyvitamin D oder Glycoside von 1,25-Dihydroxyvitamin D ist, und
wobei die leicht lösliche Calciumquelle Calciumchlorid, Calciumsulfat, Calciumpropionat, Calciumacetat, Calciumlactat oder Calciumformiat allein oder in Kombination umfasst.

8. Kombination zur Verwendung gemäß Anspruch 7, wobei die Kombination in Form eines Bolus, einer Tablette oder eines Pellets mit einer Dichte von mindestens 1,2 kg/l (g/ml) vorliegt, und die 1-alpha-hydroxylierte Vitamin-D-Verbindung und die leicht lösliche Calciumquelle über einen Zeitraum von weniger als 2 Stunden vollständig aus dem Bolus, der Tablette oder dem Pellet freigesetzt werden.

9. Kombination zur Verwendung gemäß Anspruch 7 oder Anspruch 8, wobei die 1-alpha-hydroxylierte Vitamin-D-Verbindung und die leicht lösliche Calciumquelle in einer einzigen Gesamtdosis ohne zusätzliche Dosen bereitgestellt werden, um normalere Calciumkonzentrationen im Blut zu unterstützen.

10. Kombination zur Verwendung gemäß einem der Ansprüche 7 bis 9 zur Verwendung in einem Verfahren zum Unterstützen normaler Blutcalciumkonzentrationen oder zum Reduzieren von Hypocalciämie, wobei die Kombination einem perinatalen Säugetier innerhalb von 3 Stunden nach der Geburt verabreicht wird.

11. Kombination zur Verwendung gemäß einem der Ansprüche 7 bis 10 zur Verwendung in einem Verfahren zur Verringerung von Hypocalciämie bei einem perinatalen Säugetier, bei dem das Risiko besteht, dass es zu Beginn der Laktation eine Hypocalciämie entwickelt, wobei das perinatale Säugetier eine Milchkuh ist.

12. Kombination zur Verwendung in einem Verfahren zum Erhöhen oder Normalisieren des Calciumspiegels im Blut bei einem Säugetier mit Hypocalciämie gemäß einem der Ansprüche 7 bis 11, wobei die Verabreichung der leicht löslichen Calciumquelle die passive, Vitamin-D-unabhängige, parazelluläre Absorption von Calcium fördert, um normale Calciumkonzentrationen im Blut zu unterstützen und Hypocalciämie während der ersten 6-12 Stunden nach Verabreichung der Kombination zu reduzieren, ohne eine unkompensierte metabolische Azidose bei dem perinatalen Säugetier zu induzieren, und
wobei die leicht lösliche Calciumquelle Calciumchlorid oder Calciumsulfat in einer ausreichenden Menge beinhaltet, um eine kompensierte metabolische Azidose in dem perinatalen Säugetier zu induzieren, um die Empfindlichkeit der Gewebe gegenüber Parathormon zu unterstützen, um die Calciumhomöostase innerhalb von 8 Stunden nach der Verabreichung zu verbessern.

13. Kombination zur Verwendung in einem Verfahren zum Erhöhen oder Normalisieren von Blutcalciumspiegeln bei einem Säugetier mit Hypocalciämie gemäß einem der Ansprüche 7 bis 12, wobei das perinatale Säugetier ein Wiederkäuer ist, und wobei die Verabreichung der 1-alpha-hydroxylierten Vitamin-D-Verbindung den aktiven Transport von Calcium durch das Pansen- und/oder Darmepithel stimuliert, um normale Blutcalciumkonzentrationen zu unterstützen oder Hypocalciämie von 12 bis 72 Stunden nach der Verabreichung zu reduzieren.

14. Kombination zur Verwendung gemäß einem der Ansprüche 7 bis 13, wobei das 1-alpha-hydroxylierte Vitamin D eine β-Glycosidbindung aufweist und wobei eine Quelle der 1-alpha-hydroxylierten Vitamin-D-Verbindung mit der β-Glycosidbindung *Solanum glaucophyllum, Cestrum diurnum, Trisetum flavescens* oder Kombinationen davon ist.

## Revendications

1. Composition destinée à être utilisée dans un procédé de traitement de l'hypocalcémie ou de prévention de l'hypocalcémie chez un mammifère péri-parturient présentant un risque de développer une hypocalcémie, ladite composition comprenant :
une source de calcium facilement soluble rapidement absorbable par le mammifère péri-parturient en utilisant le transport paracellulaire passif à travers l'épithélium intestinal, la source de calcium facilement soluble comprenant le chlorure de calcium, le sulfate de calcium, le propionate de calcium, l'acétate de calcium, le lactate de calcium ou le formate de calcium, seul ou en combinaison ; et
un composé de vitamine D 1-alpha hydroxylée en une quantité suffisante pour stimuler le transport actif du calcium à travers l'épithélium intestinal du mammifère péri-parturient, le composé de vitamine D 1-alpha hydroxylée étant la 1,25-dihydroxyvitamine D 1-alpha hydroxylée, la 1-alpha hydroxyvitamine D 1-alpha hydroxylée ou des glycosides 1-alpha hydroxylés de la 1,25-dihydroxyvitamine D, et
le procédé comprenant l'administration orale de la composition au mammifère péri-parturient simultanément dans les 0 à 6 heures suivant la parturition afin de maintenir des concentrations normales de calcium dans le sang chez le mammifère péri-parturient.

2. Composition destinée à être utilisée selon la revendication 1, dans laquelle la source de calcium facilement soluble est le chlorure de calcium et la source de calcium facilement soluble induit une acidose métabolique compensée dans les 8 heures suivant l'administration afin de maintenir une sensibilité normale des tissus à l'hormone parathyroïdienne.

3. Composition destinée à être utilisée selon la revendication 1 ou la revendication 2, dans laquelle le mammifère péri-parturient est un mammifère ruminant et le composé de vitamine D 1-alpha hydroxylée est présent en une quantité suffisante pour stimuler le transport actif du calcium à travers le rumen ou l'épithélium intestinal du mammifère péri-parturient.

4. Composition destinée à être utilisée selon l'une quelconque des revendications précédentes, dans laquelle la vitamine D 1-alpha hydroxylée a une liaison β-glycoside et dans laquelle une source du composé de vitamine D 1-alpha hydroxylée avec la liaison β-glycoside est *Solanum glaucophyllum, Cestrum diurnum, Trisetum flavescens,* ou des combinaisons de ceux-ci.

5. Composition destinée à être utilisée selon l'une quelconque des revendications précédentes, dans laquelle une quantité du composé de vitamine D 1-alpha hydroxylée est inférieure à 300 ug en tant que dose totale de sorte que la dose totale ne provoque pas d'inhibition significative de l'enzyme 25-hydroxyvitamine D-1-alpha hydroxylase rénale, ni n'inhibe la production endogène de 1,25-dihydroxyvitamine D, et empêche ainsi une hypocalcémie retardée de se produire 4 à 10 jours après l'administration de la composition.

6. Composition destinée à être utilisée selon l'une quelconque des revendications précédentes, dans laquelle le composé de vitamine D 1-alpha hydroxylée et la source de calcium facilement soluble sont administrés simultanément sous la forme d'un bolus, d'un comprimé ou d'une pastille ayant une densité d'au moins 1,2 kg/L (g/ml) et libérés du bolus, du comprimé ou de la pastille sur une période de temps inférieure à 2 heures.

7. Combinaison destinée à être utilisée dans un procédé d'augmentation ou de normalisation des taux de calcium dans le sang chez un mammifère atteint d'hypocalcémie, le procédé comprenant :
l'administration orale simultanée de la combinaison à un mammifère péri-parturient dans les 6 heures suivant la parturition, la combinaison comprenant séparément un composé de vitamine D 1-alpha hydroxylée et une source de calcium facilement soluble,
le composé de vitamine D 1-alpha hydroxylée étant la 1,25-dihydroxyvitamine D, la 1-alpha hydroxyvitamine D ou des glycosides de la 1,25-dihydroxyvitamine D, et
la source de calcium facilement soluble comprenant le chlorure de calcium, le sulfate de calcium, le propionate de calcium, l'acétate de calcium, le lactate de calcium ou le formate de calcium, seul ou en combinaison.

8. Combinaison destinée à être utilisée selon la revendication 7, la combinaison se présentant sous la forme d'un bolus, d'un comprimé ou d'une pastille ayant une densité d'au moins 1,2 kg/L (g/ml) et le composé de vitamine D 1-alpha hydroxylée et la source de calcium facilement soluble étant complètement libérés du bolus, du comprimé ou de la pastille sur une période de temps inférieure à 2 heures.

9. Combinaison destinée à être utilisée selon la revendication 7 ou la revendication 8, dans laquelle le composé de vitamine D 1-alpha hydroxylée et la source de calcium facilement soluble sont fournis en une seule dose totale sans aucune dose supplémentaire pour maintenir des concentrations de calcium dans le sang plus normales.

10. Combinaison destinée à être utilisée selon l'une quelconque des revendications 7 à 9, destinée à être utilisée dans un procédé de maintien de concentrations normales de calcium dans le sang ou de réduction de l'hypocalcémie, ladite combinaison étant administrée à un mammifère péri-parturient dans les 3 heures suivant l'accouchement.

11. Combinaison destinée à être utilisée selon l'une quelconque des revendications 7 à 10, destinée à être utilisée dans un procédé de réduction de l'hypocalcémie chez un mammifère péri-parturient présentant un risque de développer une hypocalcémie au début de la lactation, le mammifère péri-parturient étant une vache laitière.

12. Combinaison destinée à être utilisée dans un procédé d'augmentation ou de normalisation des taux de calcium dans le sang chez un mammifère atteint d'hypocalcémie selon l'une quelconque des revendications 7 à 11, dans laquelle l'administration de la source de calcium facilement soluble favorise l'absorption paracellulaire passive, indépendante de la vitamine D du calcium pour maintenir des concentrations normales de calcium dans le sang et réduire l'hypocalcémie pendant les 6 à 12 premières heures suivant l'administration de la combinaison et sans induire une acidose métabolique non compensée chez le mammifère péri-parturient, et
la source de calcium facilement soluble comprenant le chlorure de calcium ou le sulfate de calcium en une quantité suffisante pour induire une acidose métabolique compensée chez le mammifère péri-parturient afin de maintenir la sensibilité des tissus à l'hormone parathyroïdienne de manière à améliorer l'homéostasie du calcium dans les 8 heures suivant l'administration.

13. Combinaison destinée à être utilisée dans un procédé d'augmentation ou de normalisation des taux de calcium dans le sang chez un mammifère atteint d'hypocalcémie selon l'une quelconque des revendications 7 à 12, le mammifère péri-parturient étant un mammifère ruminant, et l'administration du composé de vitamine D 1-alpha hydroxylée stimulant le transport actif du calcium à travers le rumen et/ou l'épithélium intestinal pour maintenir des concentrations normales de calcium dans le sang ou réduire l'hypocalcémie de 12 à 72 heures après l'administration.

14. Combinaison destinée à être utilisée selon l'une quelconque des revendications 7 à 13, dans laquelle la vitamine D 1-alpha hydroxylée a une liaison β-glycoside et dans laquelle une source du composé de vitamine D 1-alpha hydroxylée avec la liaison β-glycoside est *Solanum glaucophyllum, Cestrum diurnum, Trisetum flavescens,* ou des combinaisons de ceux-ci.
